# FASCICULE DE BREVET EUROPEEN

(11) **EP 3 232 911 B1**
(45) Date de publication et mention de la délivrance du brevet: **27.10.2021**
(21) Numéro de dépôt: 15817983.8
(22) Date de dépôt: 10.12.2015
(51) Int. Cl.: A61B 5/00, A61N 1/36

(54) **SYSTÈME DE COMMUNICATION SANS FIL COMPRENANT UN NANO-DISPOSITIF ÉMETTEUR ET UN NANO-DISPOSITIF RÉCEPTEUR POUR LE TRANSFERT D'UNE PERCEPTION ET UN PROCÉDÉ DE COMMUNICATION ASSOCIÉ**
DRAHTLOSKOMMUNIKATIONSSYSTEM MIT EINER NANO-SENDER-VORRICHTUNG UND EINER NANO-EMPFÄNGER-VORRICHTUNG ZUR ÜBERTRAGUNG EINER WAHRNEHMUNG UND ZUGEHÖRIGES VERFAHREN ZUR KOMMUNIKATION
WIRELESS COMMUNICATION SYSTEM COMPRISING A NANO TRANSMITTER DEVICE AND A NANO RECEIVER DEVICE FOR THE TRANSFER OF A PERCEPTION AND AN ASSOCIATED METHOD OF COMMUNICATION

(30) Priorité: 17.12.2014 FR 1462569
(43) Date de publication de la demande: 25.10.2017
(73) Titulaire: Bull SAS, 78340 Les Clayes-sous-Bois (FR)
(72) Inventeur: ZENG EYINDANGA, Landry Stéphane, 38100 Grenoble (FR); EMMENDOERFFER, Pascal, 38160 Saint Marcellin (FR); PETRETTO, Franck, 38410 Vaulnaveys Le Bas (FR)
(74) Mandataire: IPAZ
(86) Numéro de dépôt international: PCT/FR2015/053404
(87) Numéro de publication internationale: WO 2016/097538

(56) Documents cités:
- US-A1- 2002 111 551
- US-A1- 2008 244 500
- US-A1- 2013 063 550
- SEYEDI, MIRHOJJAT; KIBRET, BEHAILU; LAI, DANIEL T.H.; FAULKNER, MICHAEL: "A Survey of Intrabody Communications for Body Area Network Applications", IEEE TRANSACTIONS ON BIOMEDICAL ENGINEERING, vol. 60, no. 8, août 2013 (2013-08), pages 2067-2079, XP002746406,

## Description

### [Domaine de l'invention]

L'invention porte sur un système permettant le transfert d'une perception à partir d'un individu dit émetteur et à destination d'un individu dit récepteur.

Plus particulièrement, l'invention consiste à reproduire à l'identique une perception d'un individu à un autre. Un tel transfert de perception peut être utile dans des applications sociologiques pour transférer des informations ou messages sensoriels entre deux individus et étudier les comportements qui en résultent, dans des applications de psychologie cognitive pour réaliser des études comparatives de la perception et analyser le temps de traitement, par l'individu récepteur, de l'information transférée ou encore dans des applications thérapeutiques qui ne sont pas comprises dans l'objet des revendications, pour transférer la perception d'une information sensorielle d'un individu émetteur sain vers un individu récepteur dont les capteurs sensoriels naturels dysfonctionnent.

On entend par la perception, l'ensemble des mécanismes de traitement des informations sensorielles extéroceptives et proprioceptives.

Les informations sensorielles extéroceptives sont les informations transmises par des extérorécepteurs situés sous la peau et dans les muqueuses et permettant de transmettre aux fibres nerveuses des informations en provenance d'une stimulation extérieure, mécanique par exemple, telle que le toucher, une pression forte, thermique, ou douloureuse par exemple. Le message nerveux transmis aux fibres nerveuses peut alors soit se poursuivre jusqu'au cortex cérébral où il devient conscient, soit être à l'origine d'un réflexe extéroceptif inconscient. Les informations sensorielles extéroceptives correspondent aux cinq sens que sont le toucher, l'ouïe, l'odorat, la vue et le goût. Enfin, les informations proprioceptives concernent la perception de la position des différentes parties du corps, grâce à de nombreux récepteurs musculaires et ligamentaires permettant de transmettre aux fibres nerveuses un message et de percevoir ainsi l'activité de son propre corps dans l'espace.

L'ensemble de ces perceptions permet à un organisme vivant d'acquérir des connaissances sur son environnement et son activité propre, d'y agir en contrôlant ses actions de manière à assurer sa survie et son adaptation à cet environnement.

En psychologie cognitive, la perception est définie comme étant la réaction d'un individu à une stimulation extérieure qui se manifeste par des phénomènes chimiques et/ou neurologiques au niveau des organes des sens et au niveau du système nerveux central, ainsi que par divers mécanismes qui tendent à confondre cette réaction à son objet par des processus tels que la représentation de l'objet, la différenciation de cet objet par rapport à d'autres objets. L'information sensorielle constitue l'ensemble des signaux qui sont le support des messages ou influx nerveux, tandis que la perception constitue l'interprétation de l'information sensorielle.

### [Art Antérieur]

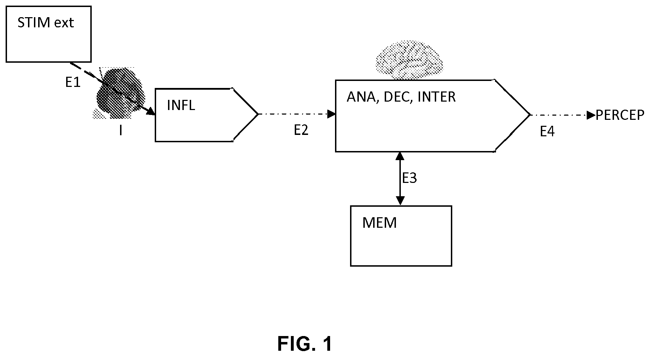

La Figure 1 représente un schéma du traitement usuel d'une perception par un individu. Dans un environnement quelconque, un individu I, pourvu de ses organes sensoriels, transforme un signal issu d'une stimulation extérieure STIMext, provoquée par un objet ou une personne par exemple, en énergie nerveuse, encore dénommée influx nerveux et référencée INFL dans la suite de la description (étape E1). Cet influx nerveux se propage, par conduction électrochimique, le long du système nerveux, grâce aux fibres nerveuses qui le transfèrent jusqu'au système nerveux central (étape E2), c'est-à-dire jusqu'au cerveau. Le cerveau analyse ANA l'influx nerveux, le décode DEC et l'interprète INTER. Il sait ainsi reconnaitre l'objet ou la personne à l'origine du stimulus, et le différencier d'un autre objet ou d'une autre personne. En réponse à la stimulation extérieure, l'individu I comprend la signification de la sensation qu'il ressent, c'est le phénomène de perception PERCEP (étape E4), et il inscrit (étape E3) ce phénomène dans sa mémoire MEM sous une ou plusieurs formes de représentations, parmi lesquelles on peut par exemple citer des représentations structurales, sémantiques, lexicales ou pragmatiques.

Il peut s'avérer intéressant de pouvoir transférer une perception d'un individu à un autre, afin de pouvoir reproduire un même comportement d'un individu à l'autre face à une même situation. Une telle reproduction de perception permettra alors de réaliser des études comparatives de la perception d'un individu à l'autre. A titre d'exemple, un médecin pourra connaitre le niveau de douleur de différents patients en réaction à un stimulus et ainsi mieux comprendre et appréhender la gestion de la douleur. Dans une autre application, une telle reproduction permettra à un individu dont les capteurs sensoriels naturels sont défaillants, de pouvoir percevoir les sensations correspondantes.

Une des solutions les plus connues pour comparer la perception d'un stimulus extérieur d'un individu à un autre est de mettre les deux individus dans la même situation physique. Cependant, cette solution ne permet pas de garantir une perception identique, car chaque individu possède sa propre façon de réagir à un même événement, si bien que chaque individu émet un influx nerveux différent lorsqu'il perçoit le stimulus extérieur. L'interprétation des sensations varient notamment en fonction de l'environnement dans lequel se trouve chaque individu. Ainsi, par exemple, un individu méditerranéen interprétera la température d'une pièce à 15°C comme étant froide alors qu'un individu sibérien interprétera cette même pièce à 15°C comme étant chaude. Par conséquent, dans ce cas de figure, la perception qui en résulte, c'est à dire l'interprétation de la sensation, n'est pas identique d'un individu à l'autre.

Le document KR101351779 décrit un nano-capteur apte à contrôler en temps réel les changements neurochimiques dans le cerveau, afin d'améliorer la qualité de vie de patients souffrant de désordres ou de maladies neurologiques.

Le document KR20080100573 décrit une interface électronique neuronale pour les commandes sensorielles et motrices du corps humain. Un micro capteur intelligent et une nano-sonde sont implantés dans le cerveau d'un patient ayant des fonctions motrices ou sensorielles anormales, afin de contrôler et de stimuler les activités nerveuses cérébrales. L'interface comprend plus particulièrement un module de contrôle et de stimulation du cerveau qui est implanté à travers le crâne pour être au contact de la zone cérébrale nerveuse. Un module de pilotage, qui est installé sur la tête du patient, fournit de la puissance, par transmission sans fil, au module implanté dans le cerveau, et communique également avec le module implanté dans le cerveau pour transmettre un signal de contrôle en fonction des zones du système nerveux à contrôler. Cette interface électronique permet donc de stimuler les zones profondes du cerveau.

Ces deux documents concernent la stimulation des zones nerveuses du cerveau afin d'améliorer la qualité de vie de personnes souffrant de maladies neurologiques. Ces demandes ne décrivent pas et ne suggèrent pas de transférer une perception d'un individu émetteur vers un individu récepteur.

Le document US2013/063550 décrit un système d'intelligence artificielle comprenant : un assistant numérique personnel (PDA), un système de capture, d'enregistrement et de mesure de l'activité cérébrale, un système de capture, d'enregistrement et de mesure de l'environnement de l'utilisateur et un système de corrélation. Le système décrit dans ce document permet d'augmenter la mémoire d'un utilisateur ou de l'améliorer. Pour cela, le système décrit permet d'identifier ce qu'une personne voit, pour permettre ensuite à un serveur central de proposer et/ou déterminer des actions en fonction de ce qui est vu. L'étape d'identification de l'environnement s'appuie sur un étalonnage répété, effectué grâce à des appareils embarqués sur une personne, le but étant de construire la représentation la plus fidèle d'une scène réelle telle que perçue par la personne. Pendant cette phase d'étalonnage, les signaux électriques, support des influx nerveux recueillis, ne sont jamais transmis tels quels. Ils sont analysés localement pour construire une représentation fidèle de la réalité qui sera ensuite transmise à un serveur central pour sauvegarde et autres analyses. Ce document se préoccupe de la représentation d'une perception, mais il ne permet pas de reproduire à l'identique la perception d'une information sensorielle, émise en réaction à un stimulus extérieur, d'un individu à un autre.

### [Problème technique]

L'invention a donc pour but de remédier aux inconvénients de l'art antérieur en proposant un système pour le transfert d'une perception à partir d'un individu dit émetteur et à destination d'un individu dit récepteur qui permette de reproduire à l'identique une même perception d'un individu à un autre, c'est-à-dire qui permette une interprétation identique d'une sensation.

### [Brève description de l'invention]

A cet effet, l'invention porte sur un système de communication sans fil pour le transfert d'une perception à partir d'un individu dit émetteur et à destination d'un individu dit récepteur, ledit système comprenant :
- un nano-dispositif émetteur, destiné à être implanté dans le système nerveux de l'individu émetteur, et un nano-dispositif récepteur destiné à être implanté dans le système nerveux de l'individu récepteur,
- ledit nano-dispositif émetteur comprenant : un nano-capteur pour intercepter un signal électrique support d'un influx nerveux émis par l'individu émetteur lorsque ce-dernier est soumis à un stimulus extérieur, un nano-processeur pour transformer le signal électrique ainsi intercepté en un signal numérique, un module d'émission / réception pour émettre ledit signal numérique vers ledit nano-dispositif récepteur et une alimentation en énergie électrique, et
- ledit nano-dispositif récepteur comprenant : un module d'émission / réception pour recevoir ledit signal numérique en provenance dudit nano-dispositif émetteur, un nano-processeur pour décoder ledit signal numérique et le transformer en un signal électrique correspondant à l'influx nerveux de l'individu émetteur, un nano-capteur apte à injecter ledit influx nerveux dans le système nerveux dudit individu récepteur et une alimentation en énergie électrique.

Ainsi, le signal électrique correspondant à l'influx nerveux injecté dans le système nerveux de l'individu récepteur est identique au signal électrique support de l'influx nerveux émis par l'individu émetteur soumis à un stimulus extérieur, de sorte que l'interprétation faite par l'individu récepteur de l'information sensorielle véhiculée par l'influx nerveux est identique à l'interprétation faite par l'individu émetteur de l'information sensorielle véhiculée par l'influx nerveux émis en réaction au stimulus extérieur auquel il a été exposé. Ainsi, la perception de l'individu récepteur est identique à la perception de l'individu émetteur.

Selon d'autres caractéristiques optionnelles du système :
- le système comprend en outre un serveur central de supervision apte à configurer les nano-dispositifs émetteur et récepteur pour qu'ils se positionnent sur une zone cible du système nerveux dédiée au traitement des informations sensorielles à l'origine de la perception à transférer, à contrôler le fonctionnement des nano-dispositifs et/ou à intervenir sur le signal numérique émis entre le nano-dispositif émetteur et le nano-dispositif récepteur,
- pour permettre son implantation sur une zone cible du système nerveux, le nano-dispositif émetteur et récepteur comprend en outre un organe de repérage de ladite zone cible, ledit organe de repérage étant choisi parmi les organes suivants: un nano-capteur biochimique, un nano-système de localisation,
- les nano-dispositifs et le serveur central de supervision communiquent entre eux par ondes radio de faible puissance, inférieure ou égale à 50mW,
- les nano-dispositifs (NDE, NDR) et le serveur central de supervision (SCS) forment un réseau de communication de type BAN, dont les fréquences d'émission des ondes sont comprises dans la bande 21MHz
- la perception à transférer est la perception d'une information sensorielle choisie parmi les informations sensorielles suivantes : l'ouïe, la vue, l'odorat, le goût ou le toucher,
- les nano-dispositifs émetteur et récepteur sont implantés dans le cerveau de l'individu respectivement émetteur et récepteur et sont positionnés sur une zone cérébrale cible dédiée au traitement des informations sensorielles à l'origine de la perception à transférer.

L'invention porte en outre sur un procédé de communication sans fil pour le transfert d'une perception à partir d'un individu dit émetteur et à destination d'un individu dit récepteur, au moyen du système de communication décrit ci-dessus, ledit procédé étant caractérisé en ce qu'il comprend les étapes consistant à :
- intercepter un signal électrique support d'un influx nerveux émis par l'individu émetteur sous l'action d'un stimulus extérieur,
- transformer ledit signal électrique en signal numérique,
- émettre ledit signal numérique à destination de l'individu récepteur,
- transformer le signal numérique reçu en signal électrique support d'un influx nerveux, destiné à être injecté dans le système nerveux dudit individu récepteur.

Selon une autre caractéristique optionnelle, le procédé comprend une étape préalable consistant à configurer les nano-dispositifs émetteur et récepteur de manière à ce qu'ils s'implantent sur une zone cible du système nerveux dédiée au traitement d'informations sensorielles à l'origine de la perception à transférer, de sorte que lorsque les nano-dispositifs sont installés dans le corps des individus respectivement émetteur et récepteur, ils repèrent et s'implantent sur ladite zone cible du système nerveux.

Enfin, l'invention porte sur une utilisation dudit système décrit ci-dessus dans des applications sociologiques, des applications de psychologie cognitive pour étudier et comprendre les comportements résultant d'une perception et analyser le traitement d'informations sensorielles par des individus.

D'autres particularités et avantages de l'invention apparaitront à la lecture de la description suivante faite à titre d'exemple illustratif et non limitatif, en référence aux Figures annexées qui représentent :
- La Figure 1, déjà décrite, un schéma du traitement usuel d'une perception par un individu,
- La Figure 2, un schéma de l'architecture globale du système selon l'invention,
- La Figure 3, un schéma d'un exemple de mise en oeuvre du système selon l'invention.

### [Description détaillée de l'invention]

On entend par « stimulus », un phénomène bioélectrique lié à l'environnement ou provenant de l'organisme, qui est capté par un récepteur naturel du corps et qui entraîne la naissance d'un influx nerveux. De cet influx nerveux, nait une sensation puis une perception.

On entend par « influx nerveux » les signaux électriques transmis par un ou plusieurs neurones face à un stimulus extérieur.

On entend par « sensation », une impression consciente produite sur les sens ou ressentie par l'organisme et causée par un stimulus sur un organe sensoriel.

On entend par « perception », la compréhension de la signification de la sensation. Le terme de perception implique une connaissance d'ordre plus élevé permettant l'interprétation de la sensation. Ce sont les connaissances qui permettent une interprétation des informations sensorielles reçues.

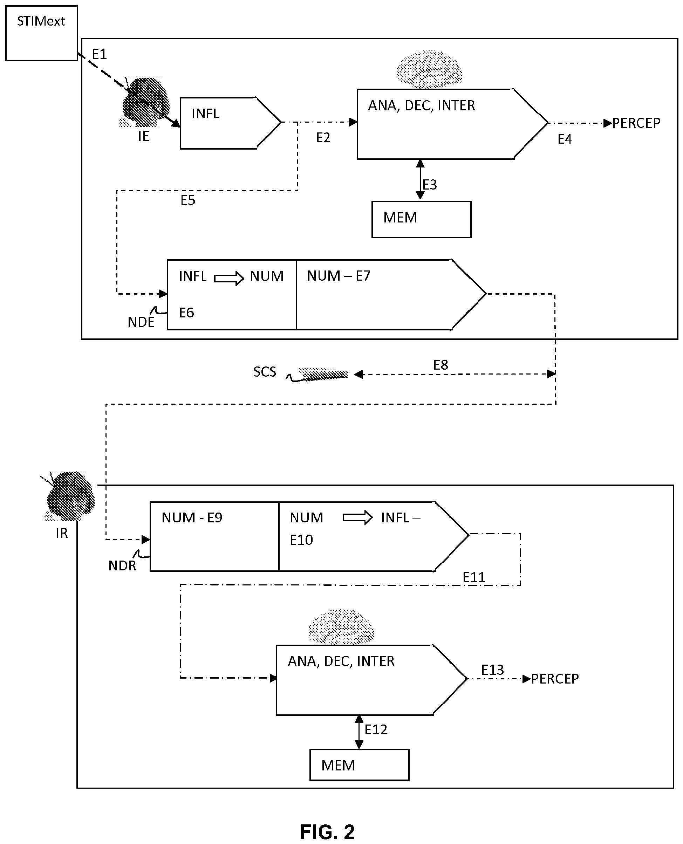

Le système de communication sans fil selon l'invention, dont l'architecture globale est schématisée sur la Figure 2, comprend deux nano-dispositifs, respectivement émetteur NDE et récepteur NDR, destinés à être implantés dans le système nerveux respectivement d'un individu émetteur IE et d'un individu récepteur IR. De tels nano-dispositifs, destinés à être implantés dans le système nerveux d'un individu, sont fabriqués à l'échelle nanométrique.

Lorsqu'un stimulus extérieur STIMext agit sur un organe sensoriel de l'individu émetteur IE, l'organe sensoriel de l'individu émetteur émet une énergie nerveuse (étape E1), encore dénommée influx nerveux INFL. Cet influx nerveux se propage (étape E2) par l'intermédiaire des fibres nerveuses du corps jusqu'au système nerveux central, c'est-à-dire le cerveau, où il est analysé ANA, décodé DEC, et interprété INTER. L'influx nerveux interprété par le cerveau de l'individu émetteur IE constitue alors la perception PERCEP (étape E4) de l'individu émetteur IE en réponse au stimulus extérieur. Ce phénomène de perception s'inscrit par ailleurs dans la mémoire MEM de l'individu émetteur (étape E3).

Pour pouvoir transférer une perception identique à un individu récepteur IR, le signal électrique support de l'influx nerveux INFL qui se propage à destination du cerveau de l'individu émetteur IE est en outre capté par le nano-dispositif émetteur NDE (étape E5). Le nano-dispositif émetteur NDE transforme alors le signal électrique support de l'influx nerveux INFL en un signal numérique NUM (étape E6), en vue de son émission, par communication sans fil, à destination du nano-dispositif récepteur NDR implanté dans le système nerveux de l'individu récepteur (étape E7).

Pour cela, le nano-dispositif émetteur NDE comprend un premier capteur capable de capter le signal électrique support de l'influx nerveux. Il comprend en outre un nano-processeur capable de transformer ce signal électrique support de l'influx nerveux INFL capté, en un signal numérique NUM.

Le nano-dispositif émetteur NDE est en outre avantageusement doté d'un module de communication sans fil, capable d'émettre un signal numérique NUM à destination d'un nano-dispositif récepteur NDR, mais aussi et de recevoir un signal en provenance notamment d'un serveur central SCS destiné à superviser son fonctionnement.

Lorsque le signal numérique NUM est reçu (étape E9) par le récepteur du module de communication sans fil du nano-dispositif récepteur NDR, le nano-processeur du nano-dispositif récepteur NDR décode le signal numérique NUM pour le transformer à nouveau en un signal électrique support d'un influx nerveux INFL (étape E10). Le nano-capteur du nano-dispositif récepteur NDR injecte ensuite le signal électrique support de l'influx nerveux, dans la zone cérébrale, de l'individu récepteur IR, dédiée au traitement des informations sensorielles correspondantes (étape E11). Le cerveau de l'individu récepteur IR analyse ANA, décode DEC et interprète INTER cet influx nerveux INFL. L'influx nerveux interprété par le cerveau de l'individu récepteur IR constitue alors la perception PERCEP de l'individu récepteur IR en réponse au stimulus extérieur ressenti par l'individu émetteur IE (étape E13). Le phénomène de perception s'inscrit par ailleurs dans la mémoire MEM de l'individu récepteur IR (étape E12).

De préférence, le signal numérique produit par le nano-dispositif émetteur NDE est transmis au nano-dispositif récepteur NDR via un réseau de communication de type BAN (acronyme anglais pour « Body Area Network »). Ce type de réseau fait par exemple appel à des standards de communication tels que Bluetooth ou Zigbee et implémente la norme IEEE 802.15.6 pour permettre à des dispositifs bio-implantés de communiquer des données grâce à une technologie sans fil, sans que les ondes émises ne produisent d'effet nocif sur le corps. Pour cela, les fréquences d'émission des ondes sont dans la bande 21MHz et les puissances d'émission sont de préférence inférieures ou égales à 50mW, et de manière encore plus préférée comprises entre 2 et 20mW.

De même, le serveur central de supervision SCS, peut communiquer, via ce réseau de communication de type BAN, avec les nano-dispositifs émetteur et récepteur, afin de les configurer préalablement à leur implantation, de contrôler leur fonctionnement, de les reconfigurer en cours d'utilisation, et/ou d'intervenir sur le signal numérique NUM en cours de transfert entre le nano-dispositif émetteur NDE et le nano-dispositif récepteur NDR.

L'installation des nano-dispositifs émetteur NDE et récepteur NDR dans le corps des individus respectivement émetteur IE et récepteur IR, pourra être réalisée de différentes manières et s'appuyer éventuellement sur des actes médicaux existants. Ainsi, par exemple, les nano-dispositifs pourront être installés par ingestion, par chirurgie ou par inoculation.

Préalablement à leur installation, les nano-dispositifs sont avantageusement configurés, au moyen du serveur central de supervision SCS, pour un transfert particulier de perception. Une telle configuration consiste à paramétrer chaque nano-dispositif, respectivement émetteur NDE et récepteur NDR, afin qu'il puisse s'implanter sur une zone précise du système nerveux, de l'individu dans lequel il est installé, dédiée au traitement d'une information sensorielle à la base de la perception à transférer.

Pour cela, le serveur central de supervision SCS permet de programmer les nano-dispositifs, et plus particulièrement leur nano-processeur pour que, une fois installé dans le corps, les nano-dispositifs exécutent les instructions de code de programmes qui ont été enregistrées au sein de leur nano-processeur afin de commander un organe de repérage embarqué pour pouvoir repérer et s'attacher à la zone cible sur laquelle ils doivent s'implanter

Pour pouvoir repérer la zone sur laquelle ils doivent s'implanter, les nano-dispositifs sont avantageusement dotés d'un organe de repérage de la zone cible. Cet organe de repérage peut par exemple prendre la forme d'un capteur bio-chimique, tel que par exemple un nano-capteur d'espèces chimiques ou biologiques à plasmons de surface capable de détecter n'importe quelle molécule dans la plus infime quantité. Un tel nanobiocapteur optique, utilisant des réseaux de nanoparticules métalliques fonctionnalisées chimiquement est connu de l'homme de l'art. Ainsi, le nano-dispositif est programmé pour s'implanter sur une zone cible particulière en fonction des informations sensorielles à capter et à interpréter. En effet, les zones du système nerveux, et notamment les zones cérébrales, ont toutes une chimie différente en fonction de leurs fonctionnalités. Par conséquent, il suffit de programmer le nano-dispositif pour que son nano-processeur active le nano-capteur biochimique afin qu'il détecte la ou les molécules identifiant une zone particulière du cerveau, qu'il la repère et s'y fixe.

Dans une variante de réalisation, on peut envisager la programmation des nano-dispositifs par des coordonnées X, Y, Z de la zone cible du système nerveux sur laquelle ils doivent se fixer. Dans ce cas les nano-dispositifs n'utilisent pas un nano-capteur biochimique comme organe de repérage, mais un nano-système de localisation in vivo.

Le serveur central de supervision SCS peut en outre communiquer avec les nano-dispositifs, au cours de leur fonctionnement, pour les contrôler, les éteindre ou les allumer, les reconfigurer, ou pour intervenir (étape E8) sur le signal numérique NUM émis entre le nano-dispositif émetteur et le nano-dispositif récepteur. Pour cela, il communique via le réseau de communication de type BAN décrit ci-dessus et implémentant la norme IEEE 802.15.

Les nano-dispositifs sont donc programmés pour s'implanter sur un emplacement fixe dans le système nerveux de l'individu, cet emplacement étant déterminé en fonction des informations sensorielles à la base de la perception à transférer.

Les nano capteurs peuvent en outre être programmés pour fonctionner pendant une durée prédéterminée au bout de laquelle ils sont programmés pour se détacher de la zone d'implantation.

Dans le cas où le nano-dispositif a été implanté par chirurgie, la dés-implantation peut être faite de la même façon.

Dans le cas d'une programmation des nano-dispositifs par détection des caractéristiques chimiques de la zone cérébrale cible, au moyen d'un nano-capteur biochimique, il convient alors, pour la désinstallation, de programmer une nouvelle destination cellulaire, au moyen du serveur central SCS de supervision par exemple, et donc indiquer au nano-dispositif sa destination « de sortie », telle que par exemple une veine, l'intestin, la vessie, etc....Pour cela, le nano-capteur biochimique embarqué comprend avantageusement deux cellules biochimiques différentes, comprenant chacune un réseau de nanoparticules métalliques fonctionnalisées chimiquement de manière différente. Une première cellule biochimique permet de repérer la zone cérébrale cible sur laquelle se fixer pour assurer le transfert de perception et une deuxième cellule biochimique est destinée à repérer une zone cible de sortie. Cette deuxième cellule biochimique est donc activée par le nano-processeur au moment où est décidée la désinstallation du nano-dispositif, c'est-à-dire lorsque la durée programmée est écoulée par exemple, ou lors d'une demande d'interruption manuelle depuis le serveur central de supervision SCS.

Dans le cas d'une programmation des nano-dispositifs par détection, au moyen d'un nano-système de localisation, des coordonnées X, Y, Z de la cellule cérébrale cible, il convient, pour la désinstallation, de reprogrammer de nouvelles coordonnées X, Y, Z d'une destination de sortie pour que les nano-dispositifs se détachent et se dirigent vers cette sortie.

Les nano-dispositifs sont autonomes énergétiquement et sont alimentés en énergie électrique au moyen de batteries par exemple. L'alimentation en énergie électrique des nano-dispositifs peut en outre être basée sur l'utilisation d'électrolytes présents dans le sang, à l'aide de deux électrodes. Un autre mode d'alimentation en énergie électrique des nano-dispositifs peut consister à obtenir de l'énergie par réaction chimique avec le sang. Dans ce dernier cas, les nano-dispositifs comprennent une réserve de produit chimique pour permettre une telle réaction.

Le nano-dispositif émetteur est programmé pour capter l'influx nerveux, le convertir en signal numérique et l'émettre vers un ou plusieurs nano-dispositifs récepteurs identifiés. De même, le (ou les) nano-dispositif(s) récepteur(s) est (sont) programmé(s) pour recevoir un signal numérique, le transformer en influx nerveux et injecter l'influx nerveux dans la zone de traitement de l'influx nerveux. Un nano-dispositif émetteur sera donc apairé avec un (ou plusieurs) autre(s) nano-dispositif(s) récepteur(s) au moment de sa programmation, pour qu'ils fonctionnent, l'un en tant qu'émetteur et l'autre (ou les autres) en tant que récepteur(s). Dans une variante de réalisation, il est possible aussi de prévoir, au moment de la configuration initiale, le cas où tous les nano-dispositifs récepteurs reçoivent les signaux émis par un ou plusieurs nano-dispositifs émetteurs mais les rejettent s'ils ne sont pas concernés.

Grâce à ce système, il est possible de transférer et reproduire une perception d'un individu à un autre. Ainsi, il est par exemple possible de transférer la perception d'une information sensorielle choisie parmi les cinq sens, d'un individu émetteur vers un individu récepteur. Les traitements perceptifs sont totalement non conscients. Pour le même stimulus extérieur, le temps de réaction et la réponse varient d'un individu à un autre et d'un essai à l'autre.

Un tel système peut être utilisé pour diverses applications. Ainsi, dans un mode de réalisation qui n'est pas couvert par l'objet des revendications, on pourra en faire un usage thérapeutique pour transférer une information sensorielle d'un individu émetteur sain vers un individu récepteur dont les capteurs sensoriels naturels correspondants sont défaillants ou dysfonctionnent. On pourra également l'utiliser pour réaliser des études, en médecine notamment, pour mieux comprendre et appréhender la gestion de la douleur par exemple. On pourra également l'utiliser en psychologie cognitive pour réaliser des études comparatives de la perception, analyser les temps de traitement suivant l'information transférée.

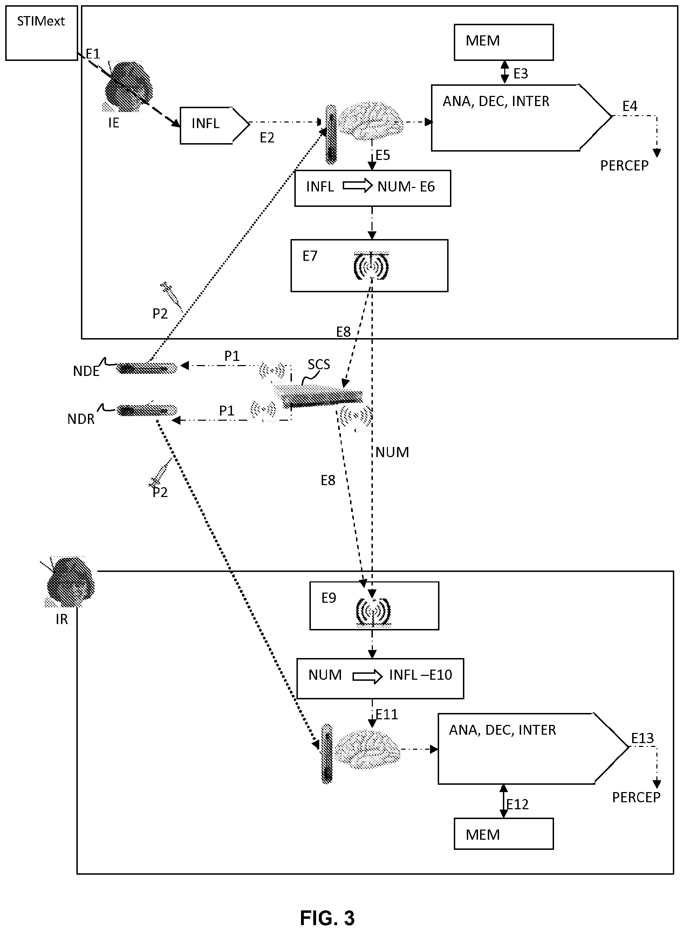

Le schéma de la Figure 3 illustre un exemple de mise en oeuvre du système selon l'invention. Selon cet exemple, le système est utilisé pour faire percevoir un son d'un individu IE à un autre IR. Ce transfert de perception auditive peut être réalisé dans le cadre d'un usage thérapeutique par exemple, pour faire entendre un son à un individu mal entendant ou sourd, mais pas seulement. Cet usage thérapeutique n'est pas compris dans l'objet des revendications. Il peut en effet s'agir de faire entendre un petit signal sonore à un individu émetteur localisé dans une pièce d'un bâtiment, et de transférer ce signal sonore à un individu récepteur, bien entendant, isolé par le port d'un casque anti-bruit ou par placement dans une pièce voisine par exemple, afin d'étudier son comportement lors de la réception de l'information correspondante et de pouvoir faire des analyses relatives au temps de traitement de l'information reçue par l'individu récepteur par exemple.

Dans ce cadre, une étape préalable au transfert de l'information de perception liée à l'audition, consiste à configurer les nano-dispositifs émetteur NDE et récepteur NDR à partir du serveur central de supervision SCS, via un protocole de communication sans fil, de type BAN tel que précédemment décrit par exemple (étape P1). Les nano-dispositifs sont ainsi programmés pour qu'ils puissent s'implanter dans la zone cérébrale cible dédiée à l'audition (aire de broadman BA 41) des individus émetteur IE et récepteur IR, grâce à un nano-capteur biochimique capable de détecter les molécules chimiques constitutives de ladite zone cible et activé par le nano-processeur de chaque nano-dispositif. Le nano-dispositif émetteur NDE est ensuite installé dans le corps de l'individu émetteur IE et le nano-dispositif récepteur NDR est installé dans le corps de l'individu récepteur IR.

Une telle installation pourra être réalisée de différentes manières et s'appuyer éventuellement sur des actes médicaux existants. Ainsi, par exemple, les nano-dispositifs pourront être installés par ingestion, par chirurgie ou par inoculation. Lorsqu'un nano-dispositif se retrouve à l'intérieur d'un corps, il exécute alors les instructions de code de programmes qui ont été enregistrées au sein de son nano-processeur, pour piloter son nano-capteur biochimique embarqué, afin que ce-dernier repère la zone cible du système nerveux dédiée au traitement des informations auditives pour lesquelles il est destiné à assurer un transfert de perception, et qu'il s'y attache (étape P2). Les nano-dispositifs sont alors prêts pour respectivement transférer et recevoir des informations.

L'individu émetteur IE perçoit un stimulus extérieur STIMext, c'est-à-dire une vibration de l'air dans cet exemple. Ce stimulus extérieur STIMext provoque l'émission d'une énergie électrique sous forme d'un influx nerveux INFL chez l'individu émetteur (étape E1), qui est transmis à la zone cérébrale dédiée à l'audition (étape E2) via les fibres nerveuses. La zone cérébrale dédiée à l'audition analyse ANA, décode DEC et traite INTER l'influx nerveux pour permettre à l'individu émetteur IE de percevoir le son PERCEP (étape E4) et d'enregistrer la perception liée au son dans sa mémoire MEM (étape E3). Le nano-dispositif émetteur NDE, implanté sur la zone cérébrale dédiée à l'audition, capte également le signal électrique support de cet influx nerveux INFL (étape E5) qu'il transforme en un signal numérique NUM (étape E6).

Le nano-dispositif émet ensuite le signal numérique NUM (étape E7), par le biais d'un réseau de communication sans fil de type BAN par exemple, à destination du nano-dispositif récepteur NDR implanté dans la zone cérébrale, de l'individu récepteur IR, destinée à l'audition. Le signal numérique NUM en provenance du nano-dispositif émetteur NDE est reçu (étape E9) par le nano-dispositif récepteur NDR.

Le serveur central de supervision SCS peut en outre intervenir sur le signal numérique en provenance du nano-dispositif émetteur NDE pour contrôler le transfert de la perception (étape E8). Ce contrôle peut par exemple consister à modifier, amplifier ou supprimer le signal numérique NUM, selon les besoins de l'étude à réaliser ou de l'application à laquelle il est destiné.

Le signal numérique NUM en provenance du nano-dispositif émetteur, éventuellement modifié par le serveur central de supervision SCS, est transformé par le nano-processeur du nano-dispositif récepteur NDR, en un signal électrique correspondant à l'influx nerveux INFL (étape E10), lequel est injecté (étape E11) dans la zone cérébrale, de l'individu récepteur IR, dédiée au traitement des informations sensorielles auditives. La zone cérébrale auditive de l'individu récepteur IR traite alors les informations sensorielles auditives reçues, pour que l'individu récepteur perçoive le son PERCEP (étape E13). Cette perception du son par l'individu récepteur est alors identique à la perception du son de l'individu émetteur. Ainsi, si l'individu émetteur IE perçoit un son grave en réponse au stimulus extérieur, l'individu récepteur IR perçoit également le son grave. Enfin, l'individu récepteur enregistre dans sa mémoire MEM le phénomène de la perception de ce son (étape E12).

## Revendications

1. Système de communication sans fil pour le transfert d'une perception à partir d'un individu dit émetteur (IE) et à destination d'un individu dit récepteur (IR), ledit système comprenant :
- un nano-dispositif émetteur (NDE), destiné à être implanté dans le système nerveux de l'individu émetteur (IE), et un nano-dispositif récepteur (NDR) destiné à être implanté dans le système nerveux de l'individu récepteur (IR),
- ledit nano-dispositif émetteur (NDE) comprenant : un nano-capteur pour intercepter un signal électrique support d'un influx nerveux (INFL), ledit influx nerveux étant émis par l'individu émetteur (IE) lorsque ce-dernier est soumis à un stimulus extérieur, un nano-processeur pour transformer ledit signal électrique intercepté par le nano-capteur en un signal numérique (NUM), un module d'émission / réception pour émettre ledit signal numérique vers ledit nano-dispositif récepteur (NDR) et une alimentation en énergie électrique, et
- ledit nano-dispositif récepteur (NDR) comprenant : un module d'émission / réception pour recevoir ledit signal numérique (NUM) en provenance dudit nano-dispositif émetteur (NDE), un nano-processeur pour décoder ledit signal numérique (NUM) et le transformer en un signal électrique correspondant à l'influx nerveux (INFL) de l'individu émetteur, un nano-capteur apte à injecter ledit signal électrique correspondant audit influx nerveux dans le système nerveux dudit individu récepteur (IR) et une alimentation en énergie électrique.

2. Système selon la revendication 1, **caractérisé en ce qu'**il comprend en outre un serveur central de supervision (SCS) apte à configurer les nano-dispositifs émetteur (NDE) et récepteur (NDR) pour qu'ils se positionnent sur une zone cible du système nerveux dédiée au traitement des informations sensorielles à l'origine de la perception à transférer, à contrôler le fonctionnement des nano-dispositifs et/ou à intervenir sur le signal numérique (NUM) émis entre le nano-dispositif émetteur et le nano-dispositif récepteur.

3. Système selon la revendication 1 ou 2, **caractérisé en ce que** pour permettre son implantation sur une zone cible du système nerveux, le nano-dispositif émetteur (NDE) et récepteur (NDR) comprend en outre un organe de repérage de ladite zone cible, ledit organe de repérage étant choisi parmi les organes suivants: un nano-capteur biochimique, un nano-système de localisation.

4. Système selon l'une des revendications 1 à 3, **caractérisé en ce que** les nano-dispositifs (NDE, NDR) et le serveur central de supervision (SCS) communiquent entre eux par ondes radio de faible puissance, inférieure ou égale à 50mW.

5. Système selon la revendication 4, **caractérisé en ce que** les nano-dispositifs (NDE, NDR) et le serveur central de supervision (SCS) forment un réseau de communication de type BAN, dont les fréquences d'émission des ondes sont comprises dans la bande 21 MHz.

6. Procédé de communication sans fil pour le transfert d'une perception à partir d'un individu dit émetteur (IE) et à destination d'un individu dit récepteur (IR), au moyen du système de communication selon l'une des revendications 1 à 5, ledit procédé ne comprenant pas d'usages thérapeutiques pour transférer une information sensorielle d'un individu émetteur vers un individu récepteur dont les capteurs sensoriels naturels correspondants sont défaillants ou dysfonctionnent, ledit procédé comprenant les étapes consistant à :
- intercepter (E5) un signal électrique support d'un influx nerveux (INFL), ledit influx nerveux étant émis par l'individu émetteur (IE) sous l'action d'un stimulus extérieur (STIMext),
- transformer (E6) ledit signal électrique en signal numérique (NUM),
- émettre (E7) ledit signal numérique (NUM) à destination de l'individu récepteur (IR),
- transformer (E10) le signal numérique (NUM) reçu en signal électrique support d'un influx nerveux (INFL), destiné à être injecté dans le système nerveux dudit individu récepteur (IR).

7. Utilisation du système selon l'une des revendications 1 à 5, dans des applications sociologiques, des applications de psychologie cognitive pour étudier et comprendre les comportements résultant d'une perception et analyser le traitement d'informations sensorielles par des individus.

## Patentansprüche

1. Drahtloses Kommunikationssystem zur Übertragung einer Wahrnehmung von einer Person, die als Sender (IE) bezeichnet wird und für eine Person bestimmt ist, die als Empfänger (IR) bezeichnet wird, das genannte System umfassend:
- eine Nano-Sendervorrichtung (NDE), die dazu bestimmt ist, in das Nervensystem der Sender-Person (IE) implantiert zu werden, und eine Nano-Empfängervorrichtung (NDR), die dazu bestimmt ist, in das Nervensystem der Empfänger-Person (IR) implantiert zu werden,
- die genannte Nano-Sendervorrichtung (NDE) umfassend: einen Nano-Sensor zum Abfangen eines elektrischen Signals, das einen Nervenimpuls (INFL) unterstützt, wobei der genannte Nervenimpuls von der Sender-Person (IE) ausgesendet wird, wenn diese letztere einem externen Reiz ausgesetzt wird, einen Nano-Prozessor zum Umwandeln des genannten, vom Nano-Sensor abgefangenen elektrischen Signals in ein digitales Signal (NUM), ein Sender-/Empfängermodul zum Senden des genannten digitalen Signals an die genannte Nano-Empfängervorrichtung (NDR), und eine Stromversorgung, und
- die genannte Nano-Empfängervorrichtung (NDR) umfassend: ein Sender-/Empfängermodul zum Empfangen des genannten digitalen Signals (NUM), das von der genannten Nano-Sendervorrichtung (NDE) kommt, einen Nano-Prozessor zum Decodieren des genannten digitalen Signals (NUM) und zum Umwandeln desselben in ein elektrisches Signal entsprechend dem genannten Nervenimpuls (INFL) der Sender-Person, einen Nano-Sensor, der in der Lage ist, das dem genannten Nervenimpuls entsprechende genannte elektrische Signal in das Nervensystem der genannten Empfänger-Person (IR) zu injizieren, und eine Stromversorgung.

2. System nach Anspruch 1, **dadurch gekennzeichnet, dass** es ferner einen zentralen Überwachungsserver (SCS) umfasst, der in der Lage ist, die Nano-Sender- (NDE) und Empfängervorrichtung (NDR) so zu konfigurieren, dass sie in einer Zielzone des Nervensystems positioniert sind, die für die Verarbeitung sensorischer Informationen am Ursprung der zu übertragenden Wahrnehmung bestimmt ist, um die Funktion der Nano-Vorrichtungen zu steuern und/oder auf das digitale Signal (NUM) zuzugreifen, das zwischen der Nano-Sendervorrichtung und der Nano-Empfängervorrichtung gesendet wird.

3. System nach Anspruch 1 oder 2, **dadurch gekennzeichnet, dass** die Nano-Sender- (NDE) und Nano-Empfängervorrichtung (NDR), um seine Implantation in einer Zielzone des Nervensystems zu ermöglichen, ferner ein Element zum Lokalisieren der genannten Zielzone umfasst, wobei das genannte Element zum Lokalisieren aus den folgenden Elementen ausgewählt ist: einem biochemischen Nano-Sensor, einem Nano-Lokalisierungssystem.

4. System nach einem der Ansprüche 1 bis 3, **dadurch gekennzeichnet, dass** die Nano-Vorrichtungen (NDE, NDR) und der zentrale Überwachungsserver (SCS) miteinander durch Funkwellen geringer Leistung von kleiner oder gleich 50 mW kommunizieren.

5. System nach Anspruch 4, **dadurch gekennzeichnet, dass** die Nano-Vorrichtungen (NDE, NDR) und der zentrale Überwachungsserver (SCS) ein Kommunikationsnetz vom Typ BAN bilden, dessen Sendefrequenzen im Band 21 MHz enthalten sind.

6. Drahtloses Kommunikationsverfahren zur Übertragung einer Wahrnehmung von einer Person, die als Sender (IE) bezeichnet wird und für eine Person bestimmt ist, die als Empfänger (IR) bezeichnet wird, mittels des Kommunikationssystems nach einem der Ansprüche 1 bis 5, wobei das genannte Verfahren keine therapeutischen Verwendungen zur Übertragung sensorischer Informationen einer Sender-Person an eine Empfänger-Person umfasst, deren entsprechende natürlichen sensorischen Sensoren fehlerhaft sind oder eine Fehlfunktion aufweisen, das genannte Verfahren umfassend die Schritte bestehend aus:
- Abfangen (E5) eines elektrischen Signals, das einen Nervenimpuls (INFL) unterstützt, wobei der genannte Nervenimpuls von der Sender-Person (IE) unter Einwirkung eines externen Reizes (STIMext) gesendet wird,
- Umwandeln (E6) des genannten elektrischen Signals in ein digitales Signal (NUM),
- Senden (E7) des genannten digitalen Signals (NUM) an die Empfänger-Person (IR),
- Umwandeln (E10) des empfangenen digitalen Signals (NUM) in ein elektrisches Signal, das einen Nervenimpuls (INFL) unterstützt, der dazu bestimmt ist, in das Nervensystem der genannten Empfänger-Person (IR) injiziert zu werden.

7. Verwendung des Systems nach einem der Ansprüche 1 bis 5 in soziologischen Anwendungen, Anwendungen der kognitiven Psychologie, um das aus einer Wahrnehmung resultierende Verhalten zu studieren und zu verstehen, und die Verarbeitung sensorischer Informationen durch Personen zu analysieren.

## Claims

1. A wireless communication system for transferring a perception from an individual called a transmitter (IE) to an individual called a receiver (IR), said system comprising:
- a nano transmitter device (NDE), intended to be implanted in the nervous system of the transmitter (IE), and a nano receiver device (NDR) intended to be implanted in the nervous system of the receiver (IR),
- said nano transmitter device (NDE) comprising: a nanosensor for intercepting an electrical signal carrying a nerve impulse (INFL), said nerve impulse being emitted by the transmitter (IE) when the latter is subjected to an external stimulus, a nanoprocessor for transforming said electrical signal intercepted by the nanosensor into a digital signal (NUM), a transmission/reception module for transmitting said digital signal to said nano receiver device (NDR) and an electrical power supply, and
- said nano receiver device (NDR) comprising: a transmission/reception module for receiving said digital signal (NUM) coming from said nano transmitter device (NDE), a nanoprocessor for decoding said digital signal (NUM) and transforming it into an electrical signal corresponding to the nerve impulse (INFL) of the transmitter, a nanosensor capable of injecting said electrical signal corresponding to said nerve impulse into the nervous system of said receiver (IR) and an electrical power supply.

2. The system according to claim 1, **characterized in that** it further comprises a central monitoring server (SCS) capable of configuring the nano transmitter (NDE) and nano receiver (NDR) devices so that they are positioned on a target area of the nervous system dedicated to the processing of sensory information at the origin of the perception to be transferred, to control the functioning of the nano devices and/or to intervene on the digital signal (NUM) emitted between the nano transmitter device and the nano receiver device.

3. The system according to claim 1 or 2, **characterized in that** to allow its implantation on a target area of the nervous system, the nano transmitter (NDE) and nano receiver (NDR) devices further comprise a device for locating said target area, said nano device locating element being chosen from the following elements: a biochemical nanosensor, a location nanosystem.

4. The system according to one of claims 1 to 3, **characterized in that** the nano devices (NDE, NDR) and the central monitoring server (SCS) communicate with each other by low-power radio waves, less than or equal to 50 mW.

5. The system according to claim 4, **characterized in that** the nano-devices (NDE, NDR) and the central supervision server (SCS) form a communication network of the BAN type, the transmission frequencies of which are within the 21 MHz band.

6. A wireless communication method for transferring a perception from an individual called a transmitter (IE) to an individual called a receiver (IR), by means of the communication system according to one of claims 1 to 5, said method not comprising therapeutic uses for transferring sensory information from a transmitter to a receiver whose corresponding natural physical sensors are faulty or malfunctioning, said method comprising the steps of:
- intercepting (E5) an electrical signal carrying a nerve impulse (INFL), said nerve impulse being emitted by the transmitter (IE) under the action of an external stimulus (STIMext),
- transforming (E6) said electrical signal into a digital signal (NUM),
- transmitting (E7) said digital signal (NUM) to the receiver (IR),
- transforming (E10) the digital signal (NUM) received into an electrical signal carrying a nerve impulse (INFL), intended to be injected into the nervous system of said receiver (IR).

7. Use of the system according to one of claims 1 to 5 in sociological applications, cognitive psychology applications, to study and understand the behaviours resulting from a perception and to analyse the processing of sensory information by individuals.
